# EUROPEAN PATENT APPLICATION

(11) **EP 1 312 685 A2**
(43) Date of publication of application: **21.05.2003**
(21) Application number: 02257873.6
(22) Date of filing: 14.11.2002
(51) Int. Cl.: C12Q 1/68

(54) **Oligonucleotide array for detecting the methylation state**

(30) Priority: 16.11.2001 JP 2001351938
(71) Applicant: NISSHINBO INDUSTRIES, INC., Chuo-ku, Tokyo (JP)
(72) Inventor: Suzuki, Osamu, c/o Nisshinbo Ind., Inc., Chiba-shi, Chiba (JP); Ichihara, Tatsuo, /o Nisshinbo Ind., Inc., Chiba-shi, Chiba (JP)
(74) Representative: Tombling, Adrian George

(57) **Abstract**

Presence or absence of methylation of C located on 5' side of G in a sample DNA comprising a target sequence containing a dinucleotide consisting of C that may be methylated and a nucleotide on the 3' side of the C (CpN dinucleotide) is determined based on a result of hybridization performed for a plurality of capture oligonucleotides immobilized on a base material and including at least an oligonucleotide having a nucleotide sequence complimentary to or identical to a nucleotide sequence corresponding to the target sequence in which C other than C in the CpN dinucleotide is replaced with T and an oligonucleotide having a nucleotide sequence complimentary to or identical to a nucleotide sequence corresponding to the target sequence in which all of C's are replaced with T's and the sample DNA in which cytosines not methylated are converted into uracils by deamination or an amplification product thereof.

## Description

### Field of the Invention

The present invention relates to an oligonucleotide-immobilized substrate and method for detecting methylation of cytosine in DNA. More precisely, the present invention provides a substrate or base material for elucidating a cause of disease invited by abnormality of genes by detecting methylated cytosines, which are present and found in a large amount in regulatory sequences for gene expression and so forth, and thereby determining prognostic treatment of the disease.

### Description of the Related Art

Methylation of DNA plays an important role in regulation of replication and expression of DNA and so forth. In eucaryocytes, methylation of DNA is frequently occurs at the 5'-position of C (cytosine) present on the 5' side of G (guanine) (henceforth referred to as "CpG dinucleotide"). In particular, many CpG dinucleotides are found in promoter regions of many genes, and such a region is called CpG island (Bird, A., Cell, 70, 5-8, 1992). Although most of these CpG islands of autosome are generally methylated, CpG islands crowdedly exist in promoter regions are not methylated (Ng, H-H. et al., Curr. Opin. Genet. Dev., 9, 158-163, 1999). It is also known that CpG islands in the promoter regions of P16 and P15, which are tumor suppressor genes, are methylated in leukemia or myeloma, and cancerization is caused by inactivation or change of expression amounts of these genes as a result of the methylation (Shu-Xia Gao et al., Leukemia Res., 24, 39-46, 2000; M. Gonzalez et al., Leukemia, 14, 183-187, 2000).

Further, it is also reported that C located on the 5' side of a nucleotide other than G may also be methylated (Gruenbaum, H., et al, Nature, 292:860-62, 1981).

Furthermore, methylation is a modification scheme of DNA responsible for epigenetic change, and in genome imprinting, there is a mechanism for methylating either one of allelic genes for suppression of expression to suppress the expression so that a gene derived from one of parents should be expressed. Therefore, if the state of methylation is changed, there may be caused onsets of viviparous fatality, hereditary diseases and so forth (Li, E., et al., Cell, 69, 915-926, 1992; Okano, M., et al., Cell, 99, 247-257, 1999; Xu, G-L., Nature, 402, 187-191, 1999).

Based on the above, it can be said that methylation of cytosine plays a significant role in control of gene expression, and change of the methylation pattern should cause diseases and so forth. Therefore, elucidation of the methylation pattern is important information for treatment and inference of prognosis of diseases.

Cytosine that is not methylated is easily converted into uracil by deamination with sodium bisulfite (Shapiro, R., et al., J. Amer. Chem., 92, 422, 1970). On the other hand, methylated cytosine is not converted into uracil even by a treatment with sodium bisulfite. Therefore, by treating DNA derived from a specimen with sodium bisulfite, DNA in which only methylated cytosines are converted into uracils can be obtained.

In order to detect methylated cytosine using a specimen DNA treated with sodium bisulfite, the following methods (1) and (2) are used.

### (1) Sequence analysis

There is a method of treating a genome derived from a specimen with sodium bisulfite, amplifying it by polymerase chain reaction (PCR) and performing sequence analysis (Marianne, F., et al., Proc. Natl. Acad. Sci. USA, 89, 1827-1831, 1992). It is also possible to clone an amplified region to perform mapping of methylation.

In this method, sequence analysis is an essential technique for mapping of methylated cytosines. However, since a regulatory region containing CpG islands has a length of 1-2 kilobases, sequencing reactions must be performed for multiple times. Therefore, it is hardly considered an easy method. Furthermore, since an expensive automatic sequencer and a dangerous radioisotope are used, it cannot be said a simple and convenient method. Moreover, it is difficult to deal many specimens in the sequence analysis.

Furthermore, it is necessary to obtain information on whether cytosines to be annealed to 3' end of primer used in the sequence analysis are modified with methyl groups beforehand. Therefore, several procedures must be required in order to elucidate whether cytosines of CpG's are methylated.

### (2) Methylation-specific PCR

A PCR primer having A (adenine) or G (guanine) at the 3' end is prepared and used to perform PCR using DNA treated with sodium bisulfite as a template (James G.H., et al., Proc. Natl. Acad. Sci. USA, 93, 9821-9826, 1996). The obtained amplification product is analyzed by electrophoresis. In this procedure, when amplification can be confirmed by using a primer having T at the 3' end, it is confirmed that cytosine at a position corresponding to the aforementioned T of the specimen DNA should be methylated. On the other hand, when amplification can be confirmed by using a primer having G at the 3' end, it is confirmed that the cytosine should not be methylated. Use of this procedure for each CpG island enables mapping of methylated cytosines.

In this procedure, however, it is necessary to prepare specific PCR primers for each CpG island. That is, at least three kinds of primers including two kinds of primers having G or A at 3' end of primers and a primer for the reverse direction are required for each CpG. Therefore, for mapping of methylated cytosines in a CpG rich regulation region, it is necessary to prepare an enormous number of PCR primers, perform PCR for an enormous number of times and analyze an enormous number of amplified PCR products by electrophoresis. Furthermore, if multiple specimens are investigated by this technique, PCR must be performed for an inestimable number of times, and therefore it is difficult to perform such investigation by this technique.

### Summary of the Invention

An object of the present invention is to provide a method for detecting methylation of C in DNA, which enables quick detection of methylated cytosines in a large number of sites in a large number of DNA samples or DNA's in a simple and convenient manner, for example, in mapping of methylated cytosines in a CpG rich regulation region and so forth.

The inventors of the present invention assiduously studied in order to achieve the aforementioned object. As a result, they found that presence or absence of methylation of C in CpG dinucleotides can be quickly detected in a simple and convenient manner by preparing a substrate on which an oligonucleotide having a nucleotide sequence complimentary to or identical to a nucleotide sequence corresponding to a target sequence in a sample DNA in which C's other than C's in CpG dinucleotides are replaced with T's, and an oligonucleotide having a nucleotide sequence complimentary to or identical to a nucleotide sequence corresponding to the target sequence in which all of C's are replaced with T's are immobilized and allowing hybridization of the oligonucleotides on the substrate and the sample DNA in which non-methylated cytosines are converted into uracils by deamination. Thus, they accomplished the present invention.

That is, the present invention provides the followings.
(1) An oligonucleotide-immobilized substrate used for detecting presence or absence of methylation of C in a sample DNA, on which multiple kinds of capture oligonucleotides are immobilized, wherein
   the sample DNA contains a target sequence containing a dinucleotide consisting of C that can be methylated and a nucleotide on the 3' side of the C (henceforth referred to as "CpN dinucleotide"),
   the capture oligonucleotides include at least an oligonucleotide having a nucleotide sequence complimentary to or identical to a nucleotide sequence corresponding to the target sequence in which C's other than C in the CpN dinucleotide are replaced with T's and an oligonucleotide having a nucleotide sequence complimentary to or identical to a nucleotide sequence corresponding to the target sequence in which all of C's are replaced with T's, and
   the presence or absence of methylation is detected based on hybridization of the sample DNA in which non-methylated C's have been converted into U's by deamination or an amplification product thereof and the capture oligonucleotides.
(2) The oligonucleotide-immobilized substrate according to (1), wherein the nucleotide on the 3' side of the C is A, G or T.
(3) The oligonucleotide-immobilized substrate according to (2), wherein the nucleotide on the 3' side of the C is G.
(4) The oligonucleotide-immobilized substrate according to any one of (1) to (3), wherein the capture oligonucleotides are immobilized via carbodiimide groups.
(5) The oligonucleotide-immobilized substrate according to any one of (1) to (4), wherein the capture oligonucleotides are immobilized as dots on a base material, and each dot has an occupied area of 0.1 cm² or less.
(6) The oligonucleotide-immobilized substrate according to any one of (1) to (5), wherein the capture oligonucleotides are oligonucleotides of 30-mer or less.
(7) The oligonucleotide-immobilized substrate according to any one of (1) to (6), wherein the sample DNA contains multiple target sequences, and capture oligonucleotides corresponding to each of the target sequences were immobilized.
(8) A method for detecting presence or absence of methylation of C in a sample DNA containing a target sequence containing a CpN dinucleotide, wherein
   multiple kinds of capture oligonucleotides immobilized on a base material and including at least an oligonucleotide having a nucleotide sequence complimentary to or identical to a nucleotide sequence corresponding to the target sequence in which C's other than C in the CpN dinucleotide are replaced with T's and an oligonucleotide having a nucleotide sequence complimentary to or identical to a nucleotide sequence corresponding to the target sequence in which all of C's are replaced with T's are hybridized with the sample DNA in which non-methylated C's have been converted into U's by deamination or an amplification product thereof, and
   the presence or absence of methylation is detected based on the result of the hybridization.
(9) The method according to claim 8, wherein the non-methylated C's have been deaminated by treatment with sodium bisulfite.

According to the present invention, methylated cytosine and cytosine not methylated in DNA can be quickly distinguished in a simple and convenient manner.

### Preferred Embodiments of the Invention

Hereafter, the present invention will be explained in detail.

The oligonucleotide-immobilized substrate of the present invention is used in order to detect presence or absence of methylation of C in a sample DNA. The sample DNA comprises a target sequence containing a dinucleotide consisting of C that can be methylated and a nucleotide on the 3' side of the C (CpN dinucleotide). The C that can be methylated usually refers to C present on the 5' side of G, however, it also refers to C present on the 5' side of a nucleotide of A, G or T, when the C can be methylated.

One target sequence or multiple target sequences may be present in the sample DNA. Further, one or more CpN dinucleotides may exist in one target sequence.

As the nucleotide on the 3' side of C, A, G and T can be mentioned, and it is preferably G.

The oligonucleotide-immobilized substrate comprises a base material and multiple kinds of capture oligonucleotides immobilized on the base material. The capture oligonucleotides include at least an oligonucleotide having a nucleotide sequence complimentary to or identical to a nucleotide sequence corresponding to the target sequence in which C's other than C's in CpN dinucleotides are replaced with T's (henceforth also referred to as "C-specific oligonucleotide") and an oligonucleotide having a nucleotide sequence complimentary to or identical to a nucleotide sequence corresponding to the target sequence in which all of C's are replaced with T's (henceforth also referred to as "T-specific oligonucleotide").

If such capture oligonucleotides as described above are allowed to hybridize with the sample DNA in which non-methylated cytosines are converted into uracils by deamination or an amplification product thereof, the C-specific oligonucleotide hybridizes to the target sequence, but the T-specific oligonucleotide does not hybridize thereto. That is, if C's in CpN dinucleotides are methylated, these C's are not converted into uracils, but C's other than C's in CpN dinucleotides are converted into uracils. On the other hand, if C's of CpN dinucleotides in the target sequence are not methylated, all of C's including those C's of CpN dinucleotides are converted into uracils. Therefore, the C-specific oligonucleotide does not hybridize to the target sequence, but the T-specific oligonucleotide hybridizes thereto. Thus, presence or absence of methylation of C's in CpN dinucleotides can be detected based on the result of the aforementioned hybridization.

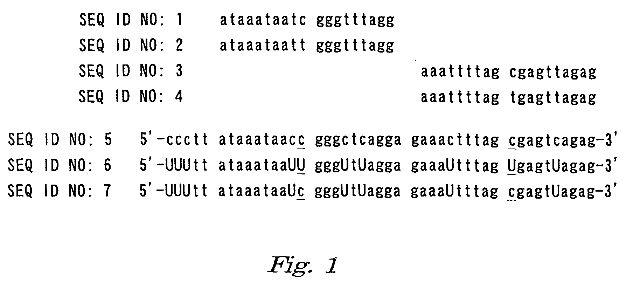

Examples of a capture oligonucleotide in which CpN dinucleotide is CpG and a target sequence are shown in Fig. 1. SEQ ID NO: 5 shows a model of nucleotide sequence of sample DNA in which C's are not deaminated. SEQ ID NO: 6 shows a model of nucleotide sequence in which all of C's of the sample DNA are deaminated and thus converted into uracils. This model represents DNA in which methylation of C does not occur. SEQ ID NO: 7 shows a model of nucleotide sequence in which C's of two CpG dinucleotides contained in the aforementioned sample DNA are conserved as they are and all of the other C's are converted into uracils. This model represents DNA in which C's of CpG dinucleotides are methylated.

SEQ ID NOS: 1 and 2 show capture oligonucleotides corresponding to the target sequence containing CpG dinucleotide on the 5' end side among two of the CpG dinucleotides. SEQ ID NO: 1 shows a C-specific oligonucleotide, and SEQ ID NO: 2 shows a T-specific oligonucleotide. SEQ ID NOS: 3 and 4 show capture oligonucleotides corresponding to the target sequence containing CpG dinucleotide on the 3' end side among two of the CpG dinucleotides. SEQ ID NO: 3 shows a C-specific oligonucleotide, and SEQ ID NO: 4 shows a T-specific oligonucleotide. The capture oligonucleotides of SEQ ID NOS: 1 and 3 and the capture oligonucleotides of SEQ ID NOS: 2 and 4 hybridize to complementary strands of DNA of SEQ ID NOS: 7 and 6, respectively.

The sample DNA may be a single-stranded DNA or a double-stranded DNA. When the sample DNA is a single-stranded DNA, the capture oligonucleotides comprises an oligonucleotide having a nucleotide sequence complementary to a nucleotide sequence of target sequence in which C's other than C's of CpN dinucleotides are replaced with T's and an oligonucleotide having a nucleotide sequence complementary to a nucleotide sequence of target sequence in which all of C's are replaced with T's. When the sample DNA is a double-stranded DNA, the capture oligonucleotides comprises a strand containing a target sequence and a strand complementary thereto, and since CpN dinucleotides are symmetrically present in each strand, the capture oligonucleotides may be the same oligonucleotides as those in the case of single-stranded DNA or oligonucleotides having nucleotide sequences complementary thereto.

Although sequence and length of the capture oligonucleotides are not particularly limited so long as difference of one nucleotide can be detected by hybridization, the capture oligonucleotides usually have a length of preferably 10- to 30-mer, more preferably 10- to 25-mer, particularly preferably 13- to 25-mer.

Oligonucleotides used as the capture oligonucleotides can be chemically synthesized by a usual solid-phase synthesis method. The aforementioned capture oligonucleotides are immobilized on a substrate. At least one pair of capture oligonucleotides corresponding to one target sequence (T-specific oligonucleotide and C-specific oligonucleotide) are immobilized, and two or more of capture oligonucleotide pairs corresponding to two or more target sequences may be immobilized on a substrate.

Each oligonucleotide is preferably immobilized at a 5' end portion or 3' end portion on a base material. The base material used for the present invention is not particularly limited so long as it can immobilize oligonucleotides by physical adsorption or chemical bonding and bear usual hybridization conditions. Specifically, there can be mentioned those insoluble in solvents used for immobilization and hybridization of oligonucleotides etc. and being in the form of solid or gel at an ordinary temperature or within a temperature range around it (e.g., 0°C to 100°C). The expression that "a base material is insoluble in a solvent" means that, after a substance having a property of bonding to oligonucleotides such as carbodiimide group is carried on the base material and oligonucleotides are immobilized on the base material as described later, the base material is substantially insoluble in various solvents including aqueous solvents and organic solvents used in various process steps when it is used as, for example, DNA chip.

Specific examples of the material of such a base material include plastics, inorganic polymers, metals, natural polymers, ceramics and so forth.

Specific examples of the plastics include polyethylene, polystyrene, polycarbonate, polypropylene, polyamide, phenol resin, epoxy resin, polycarbodiimide resin, polyvinyl chloride, polyvinylidene fluoride, polyethylene fluoride, polyimide, acrylic resin and so forth.

Specific examples of the inorganic polymers include glass, quartz crystal, carbon, silica gel, graphite and so forth.

Specific examples of the metals include gold, platinum, silver, copper, iron, aluminum, magnet, paramagnet, apatite and so forth.

Examples of the natural polymers include polyamino acids, cellulose, chitin, chitosan, alginic acid and derivatives thereof.

Specific examples of the ceramics include alumina, silica, silicon carbide, silicon nitride, boron carbide and so forth.

Examples of shape of the aforementioned base materials include, for example, film, plate, particle, molded parts (bead, strip, well of multi-well plate, strip, tube, mesh, continuous foam, membrane, paper, needle, fiber, plate, slide, cell culture vessel etc.), latex and so forth. Sizes of these are not particularly limited.

When oligonucleotides are immobilized on the base material, the oligonucleotides may be immobilized directly on the base material, or a carrier may be carried on the base material and the oligonucleotides may be immobilized on the base material via the carrier. The base material itself may have a property of bonding to the oligonucleotides, or it may be one that can immobilize the oligonucleotides via a ligand having a property of bonding to oligonucleotides. The term "carry" used herein means that the oligonucleotides do not substantially dissociate from the base material in various solvents including aqueous solvents and organic solvents used when the oligonucleotides are immobilized on the base material, the oligonucleotide-immobilized base material is used as a DNA chip and so forth.

The carrier used for the present invention may be carried by utilizing only physical adhesion or it may be chemically carried via a covalent bond or the like so long as it is carried on the aforementioned base material. Further, the carrier may be carried on the entire surface of the base material or a part thereof, as required.

Examples of the carrier include low molecular weight organic molecules, plastics, inorganic polymers, metals, natural polymers, ceramics and so forth.

Specific examples of the low molecular weight organic molecules include carbodiimide group-containing compounds, isocyanate group-containing compounds, nitrogen yperite group-containing compounds, aldehyde group-containing compounds, amino group-containing compounds and so forth.

Specific examples of the plastics include polyethylene, polystyrene, polycarbonate, polypropylene, polyamide, phenol resin, epoxy resin, polycarbodiimide resin, polyvinyl chloride, polyvinylidene fluoride, polyethylene fluoride, polyimide, acrylic resin and so forth.

Specific examples of the inorganic polymers include glass, quartz crystal, carbon, silica gel, graphite and so forth.

Specific examples of the metals include gold, platinum, silver, copper, iron, aluminum, magnet, paramagnet, apatite and so forth.

Examples of the natural polymers include polyamino acids, cellulose, chitin, chitosan, alginic acid and derivatives thereof.

Specific examples of the ceramics include alumina, silica, silicon carbide, silicon nitride, boron carbide and so forth.

Such a carrier has a highly adhesive property to the aforementioned base material, and it is adhered to the base material by utilizing this adhesive property. A typical shape of the carrier in the case where it is carried on the base material by utilizing physical adhesion property is a coated film. As the method of providing the carrier as a coated film carried on the base material, there can be used known methods such as coating using spraying, dipping, brushing, stamp, vaporization, film coater and so forth.

For example, in order to provide a resin having carbodiimide groups carried on the whole surface of glass base material, a glass base material is first immersed in a solution obtained by dissolving an amino-substituted organoalkoxysilane such as 3-aminopropyltriethoxysilane in a suitable solvent under a temperature condition of 70 to 80°C for about 2 to 3 hours, then the base material is taken out, and after the solution is washed out, the base material is dried by heating under a temperature condition of 100 to 120°C for about 4 to 5 hours. After the drying, the base material can be immersed into an appropriate solvent, and the solvent can be added with a carbodiimide resin and stirred for washing under a temperature condition of 30 to 170°C for about 12 hours. Further, it is also possible to allow the amino group of the 3-aminopropyltriethoxysilane and a functional group of nitrogen yperite group other than the oligonucleotide binding group to react by using an appropriate solvent to introduce the nitrogen yperite group on the surface of glass base material.

Introduction of various functional groups onto surfaces of such materials as described above has conventionally and generally been used, and methods therefor are known. Therefore, as also for cases where functional groups other than amino group are allowed to exist on a glass base material or the base material consists of a material other than glass, functional groups can be introduced onto the base material surface by using such known techniques.

Furthermore, some plastic base materials among those mentioned above properly have such functional groups as mentioned above. In such a case, the materials can be used as they are for the production of the base material without introducing functional groups onto surfaces thereof. Further, even such plastic base materials may be further introduced with functional groups and used for the production of the base material.

The capture oligonucleotides are immobilized on such a base material as described above at a 5' end portion or 3' end portion. The method for immobilizing oligonucleotides can suitably determined according to types of base material, functional groups on the surface of the base material, ligand and so forth.

The capture oligonucleotides may be bonded with a homopolymer consisting of three or more nucleotides on the side of an end to be immobilized on the base material. When a base material having carbodiimide groups on the surface is used, in particular, the oligonucleotides having such a homopolymer are firmly immobilized on the base material.

A known method can be used as the method of bonding such a homopolymer to ends of the oligonucleotides. For example, there can be specifically mentioned a method of synthesizing an oligonucleotide so that three or more nucleotides should be polymerized as an integral part of the oligonucleotide by using a commercially available nucleic acid synthesizer. There can also mentioned a method of binding a homopolymer to an oligonucleotide by using a chemical or enzymatic technique and so forth. The nucleobases constituting the homopolymer are selected from adenine, guanine, cytosine or thymine when the nucleic acid is DNA, or selected from adenine, guanine, cytosine or uracil when it is RNA.

The homopolymer preferably has a length of 3 to 100 nucleotides, more preferably 5 to 50 nucleotides, particularly preferably 10 to 40 nucleotides. If the number of nucleotide is 2 or less, a sufficient amount of nucleic acid cannot be immobilized on the base material. If the number of nucleotides is 101 or more, yield is markedly reduced in the nucleic acid production process.

The homopolymer may be formed from a homopolymer consisting of a certain kind of nucleotides and another homopolymer consisting of another kind of nucleotides ligated together.

Further, the 5' end of the nucleotide may be immobilized via an amino linker.

The oligonucleotides are preferably immobilized in dot-like areas. To immobilize in dot-like areas means that the areas on which the oligonucleotides are immobilized have a size sufficiently smaller than the size of the base material in such a degree that a plurality of the oligonucleotide-immobilized sites can be provided on the base material. Although shape of the dots is not particularly limited, it is generally preferably a circular shape. The oligonucleotides are usually immobilized on a plurality of sites on the base material, and the oligonucleotide-immobilized substrate is prepared as a so-called DNA array or DNA chip.

Specifically, the oligonucleotides are usually provided, for example, so as to be contained in water or a buffer so that activities of the oligonucleotides to be immobilized should be maintained during contact of the oligonucleotide and the base material for reaction. Temperature for the contact is preferably about 0 to 100°C so that the activities of the oligonucleotides should not be degraded.

The immobilization can also be attained by irradiation of electromagnetic waves such as UV rays after the contact of the oligonucleotides and the base material. Further, the immobilization can also be attained by contacting a mixture of the oligonucleotides and a known compound such as carbodiimide resin, nitrogen yperite, polyamino acids and nitrocellulose, which are chemically bonded or physically associated, with the base material. Even in such a case, the immobilization can also be attained by irradiation of electromagnetic waves such as UV rays.

In the present invention, means for providing oligonucleotides, usually in the form of water or buffer containing the oligonucleotides, in dot-like areas on the base material includes a method of utilizing a dispenser, method of utilizing a pin, method of utilizing bubble jet and so forth. However, the present invention is not limited to these. Such apparatuses for providing solutions in small amounts are commercially available, and they can be used for the present invention.

The capture oligonucleotide-immobilized base material is preferably brought into contact with an excessive amount of bovine serum albumin (BSA), casein, salmon sperm DNA or the like to block unreacted portions after the capture oligonucleotides are immobilized on the base material in dot-like areas as described above in order to prevent non-specific binding of sample DNA.

In the present invention, the sample or amplification product thereof is hybridized with the capture oligonucleotides by using the aforementioned capture oligonucleotide-immobilized substrate.

The sample DNA is not particularly limited so long as it contains CpN dinucleotides and presence of methylation of C is expected for it, and DNA of various organisms may be used depending on the purpose. The sample DNA is prepared from microbial cell, various tissues of animals or plants. The sample DNA can be prepared in the same manner as usual preparation of DNA from cells. Further, although DNA prepared from cells can also be used as it is as the sample DNA, DNA of which target sequence or a region including the target sequence is amplified by PCR method or the like may also be used.

In the conventional methylation-specific PCR, at least three kinds of primers are required for one CpN. However, according to the present invention, a region containing multiple CpN's can be amplified with two kinds of primers. Further, in the methylation-specific PCR, a primer is designed so that its 3' end should correspond to cytosine of CpN. Therefore, when many CpN sequences exist as in CpG island, methylation of CpN can be detected only for a relatively narrow region. On the other hand, according to the present invention, a primer may be designed so that C of CpN should not correspond to the 3' end, and thus PCR amplification is possible irrespective of methylation of C in CpN.

As for the sample DNA or its amplification product, C's not methylated are converted into uracils by deamination prior to the hybridization. This deamination of non-methylated C's can be performed by treating the sample DNA with bisulfite such as sodium bisulfite. Specifically, a solution containing the sample DNA is added with sodium bisulfite (pH 5) at a concentration of 2.5 M to 3.0 M and heated at 50°C for 16 hours (J.G. Herman et al., Proc. Natl. Acad. Sci. USA, 93, 9821-9826, 1996). After the reaction with heating, the sample DNA is adsorbed on a silica-based filter for purification to remove the sodium bisulfite. After the removal, the sample DNA is collected from the silica-based filter and concentrated by ethanol precipitation or the like.

Although specific procedure of the hybridization is not particularly limited, it can be performed by, for example, immersing a substrate on which the capture oligonucleotides are immobilized in a solution containing the sample DNA, or spotting a solution containing the sample DNA onto areas where the capture oligonucleotides are immobilized. Further, areas where the capture oligonucleotides are immobilized may be covered with frames so that the frames should include the areas, and a solution containing the sample DNA may be poured into the frames.

The solution is not particularly limited so long as it allows hybridization of DNA, and various buffers can be used for it. For example, Tris-HCl having pH of about 6.5 to 8 can be mentioned. Usually, the solution is heated to a temperature at which base pairs formed in molecules of DNA or oligonucleotides dissociate, e.g., 90 to 100°C, and then adjusted to a temperature at which the capture oligonucleotides and a target sequence in the sample nucleic acid anneal. Specifically, this temperature is preferably 5 to 80°C in general, although it depends on length of the capture oligonucleotides.

Although the method for detecting the hybridization is not particularly limited, it can be detected by, for example, labeling the sample nucleic acid with a labeling substance beforehand and detecting the label after the hybridization and washing of the substrate. Although the labeling substance is not particularly limited, examples thereof include substances usually used for detection of hybridization, for example, radioisotopes, fluorescent dyes, biotin, haptens, antigens and so forth. When biotin is used as a labeling substance, for example, a conjugate of a protein specifically binding to biotin (avidin or streptoavidin) and an enzyme chemically bonded to biotin can be bound after the hybridization operation, and a reaction can be performed by adding a compound that is degraded with the enzyme to form a dye that can deposit to readily detect presence or absence and location of hybridization.

### Examples

Hereafter, the present invention will be explained more specifically with reference to the following example. In the following example, investigation was performed for explaining the present invention by using a sequence of human NF-IL6 gene (GenBank Accession: AF350408) as a model system. However, application of the present invention is not limited only to this gene.

### <1> Synthesis of oligonucleotides

Oligonucleotides having an amino group or hydroxyl group at the 5' end were synthesized by using an oligonucleotide synthesizer (Perkin-Elmer Applied Biosystems), deprotected and dried in a conventional manner. These dried oligonucleotides were dissolved in 10 mM Tris-HCl (pH7.5), 1 mM EDTA buffer to prepare 100 pmol/µL oligonucleotide solutions. Sequences of the synthesized oligonucleotides are shown as SEQ ID NOS: 1-4 in Sequence Listing.

SEQ ID NO: 1 5'-cctaaaccc gattatttat-3'

SEQ ID NO: 2 5'-cctaaaccc aattatttat-3'

SEQ ID NO: 3 5'-ctctaactcg ctaaaattt-3'

SEQ ID NO: 4 5'-ctctaactca ctaaaattt-3'

### <2> Spotting of oligonucleotide on substrate (case of using oligonucleotide having an amino group at 5' end)

A solution of oligonucleotide having an amino group at 5' end in a volume of 10 µL was mixed with 10 µL of a microspotting solution (TeleChem International Inc.) and dispensed on a microtiter plate (Greiner Laboratory Ltd.). Silanized slide glass (Matsunami Glass Ind., Ltd.) was mounted on a predetermined position on a spotting machine, and the spotting machine was operated. After completion of the spotting, the slide glass was exposed to steam from hot water for several seconds. Then, the slide glass was irradiated with an ultraviolet ray of 30 mJ. After further exposure to steam for several seconds, the slide glass was placed on a hot plate to remove moisture. The slide glass was rinsed with 0.1% sodium dodecylsulfate aqueous solution and then rinsed with distilled water. The slide glass was immersed in 100 mM Tris-HCl (pH 7.5), 100 mM NaCl, 0.1% Triton X-100 containing 3% BSA (bovine serum albumin) at room temperature for 30 minutes for blocking. Then, the slide glass was dried at room temperature and washed with 10 mM Tris-HCl (pH 7.5), 1 mM EDTA buffer. The slide glass was dried again at room temperature and stored in a cold dark place in a dried state until use.

### <3> Spotting of oligonucleotide on substrate (case of using oligonucleotide having OH group at 5' end)

A solution of oligonucleotide having a hydroxyl group at 5' end in a volume of 10 µL was mixed with 10 µL of a spotting solution (2 M sodium chloride aqueous solution) and dispensed on a microtiter plate (Greiner Laboratory Ltd.). Slide glass having carbodiimide groups was disposed on a predetermined position on a spotting machine, and the spotting machine was operated. After completion of the spotting, the slide glass was immersed in 100 mM Tris-HCl (pH 7.5), 100 mM NaCl, 0.1% Triton X-100 containing 3% BSA (bovine serum albumin) at room temperature for 30 minutes for blocking. Then, the slide glass was dried at room temperature and washed with 10 mM Tris-HCl (pH 7.5), 1 mM EDTA buffer. The slide glass was dried again at room temperature and stored in a cold dark place in a dried state until use.

### <4> Preparation of nucleic acid probe

An oligonucleotide having a hydroxyl group at the 5' end was synthesized by using an oligonucleotide synthesizer (Perkin-Elmer Applied Biosystems), deprotected and dried in a conventional manner. The dried oligonucleotide was dissolved in 10 mM Tris-HCl (pH 7.5), 1 mM EDTA buffer to prepare a 100 pmol/µL oligonucleotide solution. Sequence of the synthesized oligonucleotide is shown as SEQ ID NO: 5 in Sequence Listing.

The oligonucleotide shown as SEQ ID NO: 5 in an amount of 10 µg was methylated by using CpGenome DNA Modification Kit (Intergen) to prepare an oligonucleotide in which all of C's in the sequence were converted into U's (SEQ ID NO: 6). Further, an oligonucleotide having the sequence shown as SEQ ID NO: 6 was synthesized by using a DNA synthesizer. Moreover, an oligonucleotide in which only C's of CpG's were methylated and not converted into U's and other C's were all converted into U's (SEQ ID NO: 7) was also synthesized by using a DNA synthesizer. Furthermore, after the synthesis, biotin was introduced into the 5' end of the oligonucleotides in a conventional manner.

SEQ ID NO: 5

5'-ccctt ataaataacc gggctcagga gaaactttag cgagtcagag-3'

SEQ ID NO: 6

5'-UUUtt ataaataaUU gggUtUagga gaaaUtttag UgagtUagag-3'

SEQ ID NO: 7

5'-UUUtt ataaataaUc gggUtUagga gaaaUtttag cgagtUagag-3'

### <5> Hybridization

Each of the nucleic acid probes of SEQ ID NOS: 5-7 prepared in the above aforementioned <4> was taken in a volume of 5 µL (1 pmol), placed in a 1.5 mL-volume tube, added with 20 µL of UniHyb Hybridization solution (Telechem International Inc.) and mixed to prepare a hybridization solution. This solution was treated by heating at 70°C for 5 minutes and placed on ice for 5 minutes. This nucleic acid probe in a volume of 10 µL was placed on each of the substrates on which the oligonucleotides were spotted prepared in the above <2> and <3>, and cover glass was placed on them.

The substrate was put into Hybridization Cassette (Telechem International Inc.), immersed into water at 42°C in a tank and left for 1 hour under light shielding. The substrate was taken out from Hybridization Cassette and immersed in 2 x SSC (0.75 M NaCl, 0.075 M sodium citrate) at room temperature to remove the cover glass. An operation of immersing the substrate in 0.3 x SSC at 45°C for 5 minutes was performed twice. The substrate was finally transferred into a solution of 2 x SSC.

### <6> Chemical coloration reaction

The substrate was taken out from a vessel, moisture on the area in which the oligonucleotides were not immobilized was wiped off with paper towel, and 200 µL of Block Ace solution (Dainippon Pharmaceutical Co., Ltd.) was placed on the oligonucleotide-immobilized area. After the substrate was left at room temperature for 30 minutes, the solution was sucked up with a dispenser, and 200 µL of a solution obtained by adding one drop each of avidin DH and biotinylated peroxidase (Vector Laboratories, Inc.) to 5 mL of TBST solution (50 mM Tris-HCl (pH 7.5), 0.15 M NaCl. 0.05% Tween 20) and mixing them was placed on the oligonucleotide-immobilized area of the substrate and left at room temperature for 30 minutes. The solution was sucked up with a dispenser, and the substrate was transferred to a vessel containing TBST solution and washed at room temperature for 5 minutes with shaking. The solution was once discarded, and fresh TBST solution was added to the vessel to wash the substrate at room temperature for 5 minutes with shaking. The substrate was taken out from the vessel, and moisture on the area in which the oligonucleotides were not immobilized was wiped off with paper towel. On the oligonucleotide-immobilized area, 200 µL of TMB (Vector Laboratories, Inc.) was placed, and a reaction was allowed at room temperature for 20 minutes. Then, the substrate was washed with deionized water to terminate the enzymatic reaction.

### <7> Detection of chemical coloration

The coloring image on the substrate were captured and stored as a TIFF image in a computer by using an OA scanner (NEC Multireader 600SR) and Photoshop Ver. 5.0 (Adobe Systems). The result is shown in Fig. 2. In this image, pigmentation formed by chemical coloration, if present, indicated that the oligonucleotide immobilized on the substrate (capture oligonucleotide) and the nucleic acid probe were fully complementary to each other and thus a duplex was formed.

## Claims

1. An oligonucleotide-immobilized substrate used for detecting presence or absence of methylation of C in a sample DNA, on which multiple kinds of capture oligonucleotides are immobilized, wherein
the sample DNA contains a target sequence containing a dinucleotide consisting of C that can be methylated and a nucleotide on the 3' side of the C (henceforth referred to as "CpN dinucleotide"),
the capture oligonucleotides include at least an oligonucleotide having a nucleotide sequence complimentary to or identical to a nucleotide sequence corresponding to the target sequence in which C's other than C in the CpN dinucleotide are replaced with T's and an oligonucleotide having a nucleotide sequence complimentary to or identical to a nucleotide sequence corresponding to the target sequence in which all of C's are replaced with T's, and
the presence or absence of methylation is detected based on hybridization of the sample DNA in which non-methylated C's have been converted into U's by deamination or an amplification product thereof and the capture oligonucleotides.

2. The oligonucleotide-immobilized substrate according to claim 1, wherein the nucleotide on the 3' side of the C is A, G or T.

3. The oligonucleotide-immobilized substrate according to claim 2, wherein the nucleotide on the 3' side of the C is G.

4. The oligonucleotide-immobilized substrate according to any one of claims 1-3, wherein the capture oligonucleotides are immobilized via carbodiimide groups.

5. The oligonucleotide-immobilized substrate according to any one of claims 1-4, wherein the capture oligonucleotides are immobilized as dots on a base material, and each dot has an occupied area of 0.1 cm² or less.

6. The oligonucleotide-immobilized substrate according to any one of claims 1-5, wherein the capture oligonucleotides are oligonucleotides of 30-mer or less.

7. The oligonucleotide-immobilized substrate according to any one of claims 1-6, wherein the sample DNA contains multiple target sequences, and capture oligonucleotides corresponding to each of the target sequences were immobilized.

8. A method for detecting presence or absence of methylation of C in a sample DNA containing a target sequence containing a CpN dinucleotide, wherein
multiple kinds of capture oligonucleotides immobilized on a base material and including at least an oligonucleotide having a nucleotide sequence complimentary to or identical to a nucleotide sequence corresponding to the target sequence in which C's other than C in the CpN dinucleotide are replaced with T's and an oligonucleotide having a nucleotide sequence complimentary to or identical to a nucleotide sequence corresponding to the target sequence in which all of C's are replaced with T's are hybridized with the sample DNA in which non-methylated C's have been converted into U's by deamination or an amplification product thereof, and
the presence or absence of methylation is detected based on the result of the hybridization.

9. The method according to Claim 8, wherein the non-methylated C's have been deaminated by treatment with sodium bisulfite.


Sequence Listing

<110> Nisshinbo Industries, Inc.

<120> Oligonucleotide-Immobilized Substrate for
Detecting Methylation

<130> OP1390

<140>
<141> 2002-11-

<150> JP 2001-351938
<151> 2001-11-16

<160> 7

<170> PatentIn version 3.0

<210> 1
<211> 19
<212> DNA
<213> Artificial/Unknown

<220>
<221> misc_feature
<222> ()..()
<223> Description of Artificial Sequence: capture
oligonucleotide

<400> 1
cctaaaccc gattatttat 19

<210> 2
<211> 19
<212> DNA
<213> Artificial/Unknown

<220>
<221> misc_feature
<222> ()..()
<223> Description of Artificial Sequence: capture
      oligonucleotide

<400> 2
cctaaaccca attatttat                                                    19

<210> 3
<211> 19
<212> DNA
<213> Artificial/Unknown

<220>
<221> misc_feature
<222> ()..()
<223> Description of Artificial Sequence: capture
      oligonucleotide

<400> 3
ctctaactcg ctaaaattt                                                    19

<210> 4
<211> 19
<212> DNA
<213> Artificial/Unknown

<220>
<221> misc_feature
<222> ()..()
<223> Description of Artificial Sequence: capture
      oligonucleotide

<400> 4
ctctaactca ctaaaattt                                                    19

<210> 5
<211> 45
<212> DNA
<213> Artificial/Unknown

<220>
<221> misc_feature
<222> ()..()
<223> Description of Artificial Sequence: probe
      oligonucleotide

<400> 5
cccttataaa taaccgggct caggagaaac tttagcgagt cagag                45

<210> 6
<211> 45
<212> DNA
<213> Artificial/Unknown

<220>
<221> misc_feature
<222> ()..()
<223> Description of Artificial Sequence: probe
      oligonucleotide

<400> 6
uuuttataaa taauugggut uaggagaaau tttagugagt uagag                45

<210> 7
<211> 45
<212> DNA
<213> Artificial/Unknown

<220>
<221> misc_feature
<222> ()..()
<223> Description of Artificial Sequence: probe
      oligonucleotide

<400> 7
uuuttataaa taaucgggut uaggagaaau tttagcgagt uagag                45